Europäisches Patentamt

European Patent Office

Office européen des brevets

(11) Numéro de publication : **0 518 827 A2**

# (12) DEMANDE DE BREVET EUROPEEN

(21) Numéro de dépôt : **92830225.6**

(22) Date de dépôt : **14.05.92**

(51) Int. Cl.⁵ : **G01N 33/543,** G01N 35/00

(30) Priorité : **13.06.91 IT RM910414**

(43) Date de publication de la demande :
**16.12.92 Bulletin 92/51**

(84) Etats contractants désignés :
**AT BE CH DE DK ES FR GB LI NL SE**

(71) Demandeur : **Cosimi, Simonetta**
**via Margherita n. 1**
**I-34126 Trieste (IT)**

(72) Inventeur : **Recinelli, Nevio**
**via Doberdò 95**
**I-00054 Fiumicino (Roma) (IT)**
Inventeur : **Cosimi, Simonetta**
**via Margherita n. 1**
**I-34126 Trieste (IT)**

(54) **Système (réactif et instrument) pour determiner de facon complètement automatique la présence d'immunoglobulines E totales ou dirigée contre allergènes, ou d'autres molécules dans des échantillons aqueux.**

(57)    En ce qui concerne le dosage allergologique in vitro, jusq'à maintenant il n'est pas totalement automatique ni bien reproductible. La présente invention permet de faire exécuter de manière totalement automatique à un appareillage spécial le dosage des IgE totales et spécifiques et d'obtenir des résultats bien reproductible grace aux speciales chambres de reaction (15, 16) et à la courbe de référence pour chaque différent groupe de molécules, compris dans le Système. Le Système est applicable aussi à d'autres determinations de molécules dans des échantillons aqueux.

FIG. 6

La présente invention concerne un Système complet, nommé "ENEAS Bioallergy Nouveau Système Efficace en Allergie" utilisé pour mesurer de facon complètement automatique, grace à la séparation en phase solide, les analytes présents en solution, en particulier les marqueurs de pathologies allergiques.

Les analytes présents dans les fluides biologiques sont généralement déterminés grace à de spécifiques réactions immunologiques entre un anticorps et un antigéne qui forment un immuno-complexe. Le réactif qui réagit de facon spécifique avec l'analyte est marqué afin de déterminer et de mesurer la concentration de l'analyte meme. Le marquage peut etre fait avec des émetteurs radioactifs, fluorescents, chimioluminescents ou bien avec des enzymes. En ce dernier cas le signal relatif à l'enzyme doit etre rendu évident grace à l'adjonction d'un substrat convenable qui doit modifier son état de facon à rendre le signal mémé décelable (par exemple en changeant la couleur ou l'état énergétique).

Pour faciliter la séparation entre l'analyte qui a réagi et celui qui n'a pas réagi (et qui donc n'est pas spécifiquement en relation avec le test effectué) on utilise des phases solides différentes. Le lien du réactif spécifique à la phase solide choisie permet de séparer facilement l'immunocomplexe qui se forme entre le réactif spécifique et l'analyte. (R. Rose et al., Manual of Clinical Immunology, 2nd ed. American Society for Microbiology, Washington, pp.327-429,775-849 (1980) et A. Voller et al., Immunoassay for the 80's, University Park Press, Baltimore (1981).

En ce qui concerne les dosages allergologiques in vitro, il est possible d'utiliser un grand nombre de phases solides, comme par exemple disques en papier, petites sphères en plastique, micro-puits à microplaque, éprouvettes, capsules en cellulose et ainsi de suite. Si la phase solide est mobile, il est impossible de l'identifier et il existe le risque de l'emporter en cours de réaction sans le vouloir, ce qui rend impossible mener l'analyse à bonne fin. Si la phase solide est de type fixe le volume de réaction est très important (de 50 à 200 ul).

Quelques unes des phases solides susmentionnées permettent d'automatiser certains passages du procédé, mais aucune d'elles ne consent l'automation complète de tout le procédé.

Bien que les méthodes pour le dosage in vitro de molécules étant en relation avec des pathologies de type allergique soient très diffusées (en particulier le dosage in vitro des immunoglobulines de type E) et qu'elles fournissent des résultats satisfaisants, jusqu'à maintenant elles n'ont permis ni de faire exécuter de manière totalement automatique à un appareillage spécialement concu toutes les phases du procédé, ni d'obtenir des résultats ayant reproductibilité, précision et soin comparables à ceux qu'on obtient avec d'autres types de test. Cela se produit en particulier parce que les courbes de référence sont prépareés pour un seul type de molécule, tandis qu'on en analyse beaucoup d'autres avec des caractéristiques de réaction complètement différentes. Notre invention vise à améliorer ces deux propriétés.

Conformément à la présente invention, le problème de l'automation est résolu à l'aide d'un instrument capable d'exécuter toutes les phases du procédé, grace à de spéciaux récipients de dimension capillaire en forme de "U" auxquels on colle un réactif qui réagit de facon spécifique avec l'analyte présent dans le fluide examiné.

Cet instrument est à mémé de distribuer les échantillons analysés dans la chambre de réaction, attendre le temps nécessaire pour que la réaction ait lieu, enlever grace à un lavage approprié, par distribution d'un canal de la chambre à "U" et par aspiration de l'autre coté de la solution qui n'a pas réagi, dispenser le réactif appelé marqueur, attendre le temps nécessaire pour que la deuxième réaction ait lieu, répéter les procédés de lavage de la solution qui n'a pas réagi, distribuer la solution indicateur appropriée, attendre le développement de la réaction et lire la variation colorimétrique ou énergétique qui se présente. Le problème relatif à la reproductibilité et à la précision des données est résolu grace à l'automation mémé et à la spéciale chambre de réaction qui permet d'obtenir des performances qualitatives spéciales.

D'un autre coté le problème du soin est résolu grace à la standardisation effectuée pour chaque groupe de molécules analysées; cette standardisation est mémorisée par l'instrument et peut etre controlée à nouveau par le re-calibrage a discrétion de l'usager suivant la bonne pratique de laboratoire.

Le système ENEAS suivant cette invention permet d'atteindre des résultats qualitativement meilleurs grace à la possibilité d'obtenir les résultats à l'aide d'une courbe de référence pour chaque groupe homogéne de molécules et mémé de réduire l'emploi du personnel puisque l'entier procédé est exécuté de facon totalement automatique par l'instrument objet de l'invention, avec les réactifs spécifiques.

Le Système (ENEAS Bioallergy Nouveau Système Efficace en Allergie) comprend:

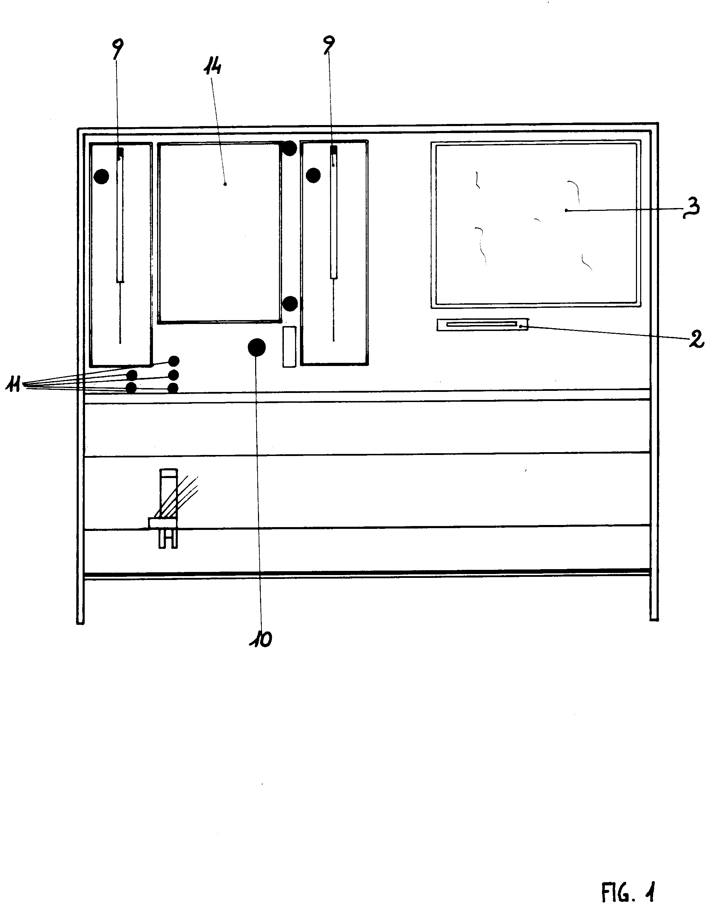

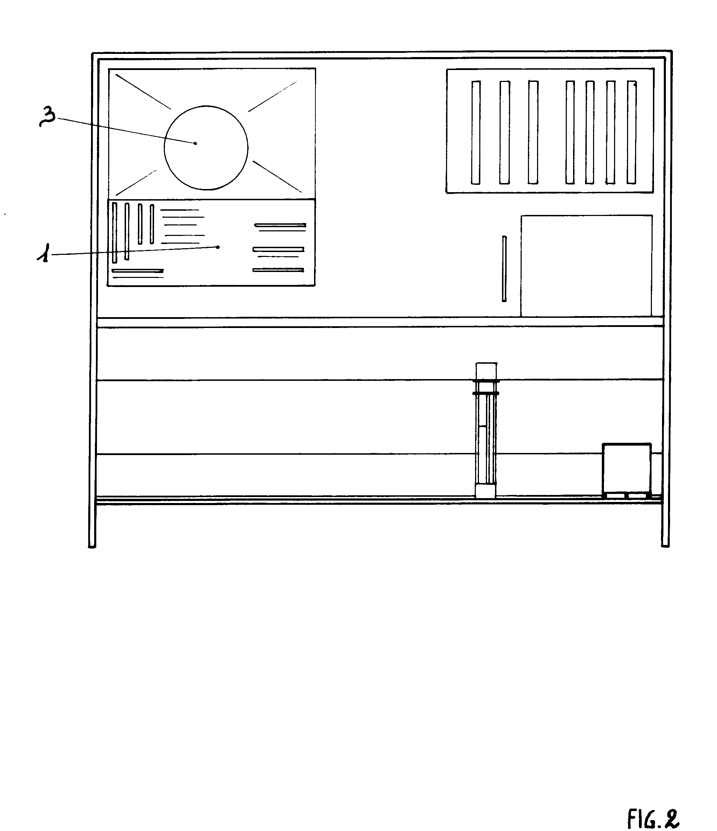

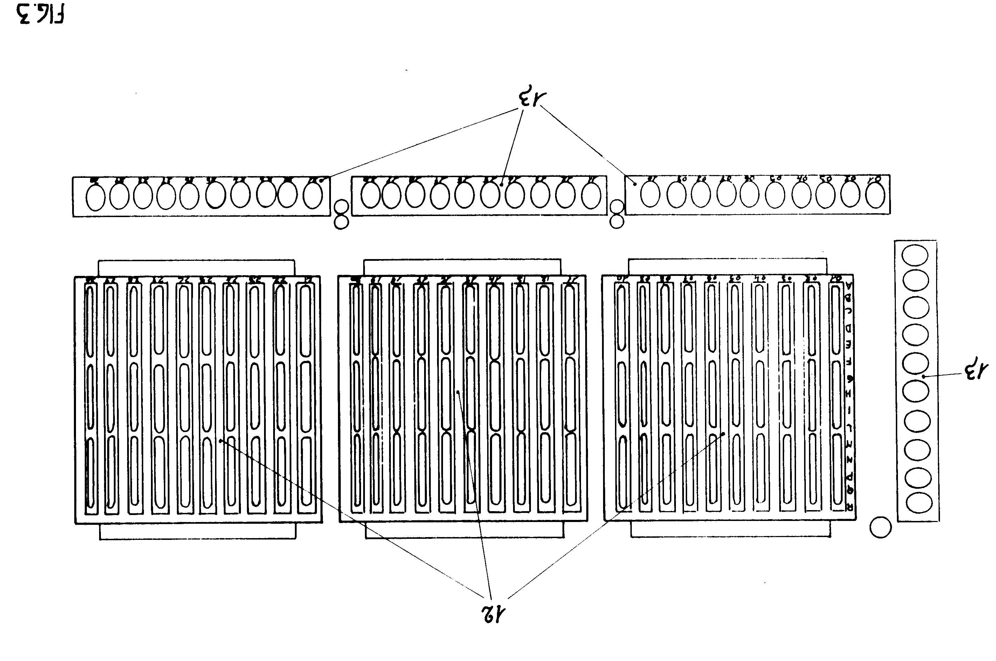

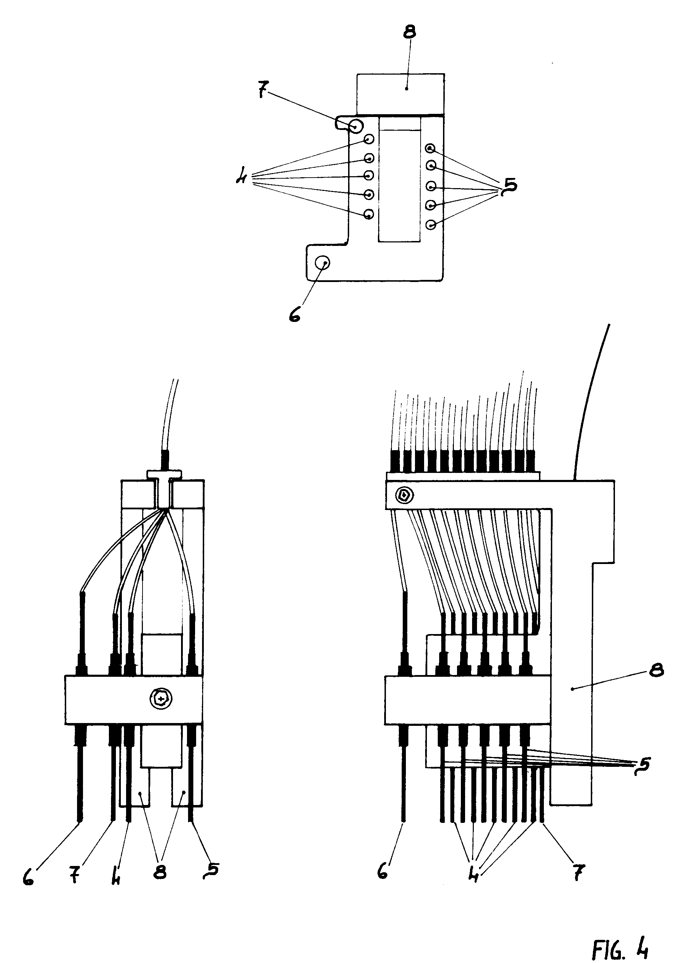

Instrument Automatique pour l'exécution automatique du procédé (Figures 1, 2, 3, 4);

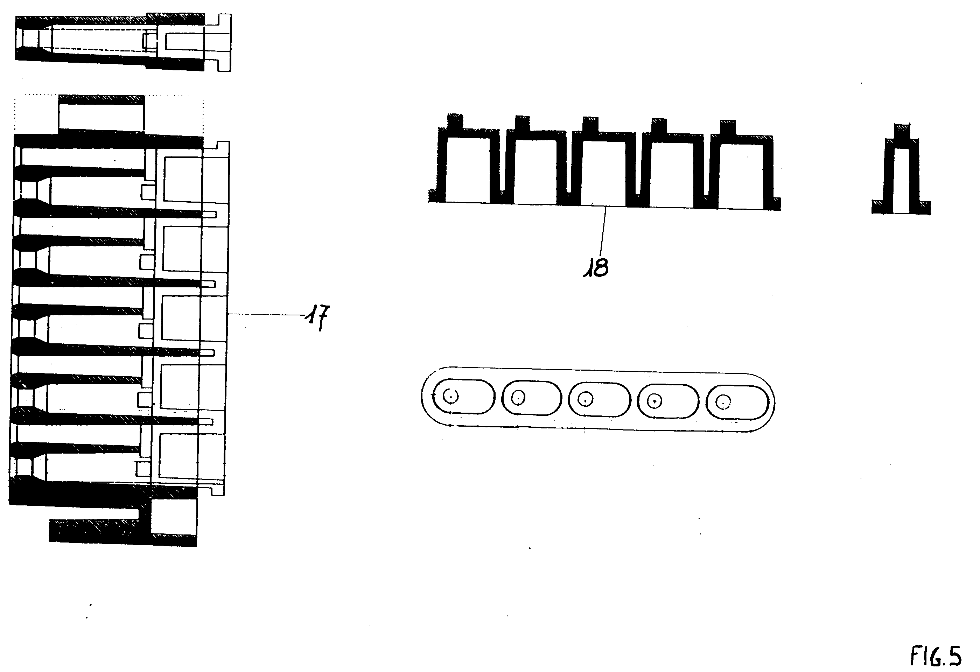

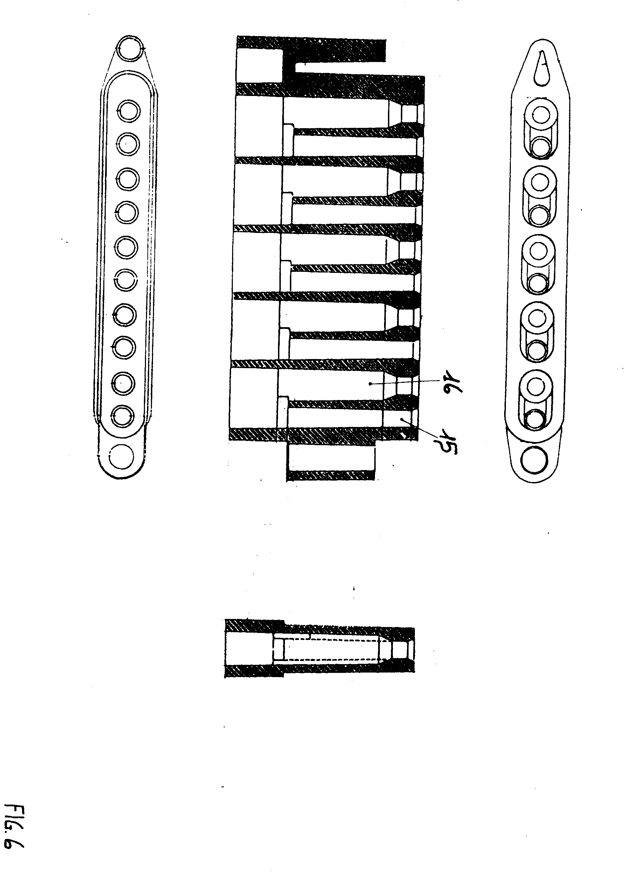

Chambres Réactives, spéciales chambres de réaction (Figures 5, 6);

Solutions réactives, réactifs pour la détermination de la réaction;

Référence, standard de référence pour chaque différent groupe de molécules;

Calibreur, sérum de controle pour le recalibrage de la courbe standard;

CQ, Programme de controle de Qualité Interlaboratoire en Allergologie.

L'instrument pour l'exécution automatique du procédé d'analyse est un appareil compact, comprenant:

un ordinateur AT avec disque rigide (1), un drive pour disques de 3 pouces et 1/2 (2), clavier et écran (3); l'ordinateur utilise un programme dédié pour la gestion des parties mécaniques de l'instrument mémé, l'exécution du procédé et la gestion des archives patients;

un bras mécanique pourvu de 10 aiguilles pour le lavage des chambres de réaction, 5 aiguilles pour la distribution de la solution de lavage (4) et 5 aiguilles pour l'aspiration de la solution de lavage (5), une aiguille pour la distribution de l'échantillon et du réactif marqueur (6), et une aiguille pour la distribution du réactif de détermination indicateur (7);

une tete optique pour la lecture du résultat final directement dans les chambres de réaction (8), tete qui est ancrée au bras mécanique susmentionné;

deux seringues da 5 ml, reliées à deux dilueurs pour la distribution des solutions de réaction (9);

une pompe péristaltiques pour la distribution de la solution de lavage (10);

cinq valves (11) et une pompe à vide ( ou bien une pompe péristaltique) pour l'aspiration des solutions de réaction et lavage et les opérations de décharge des tuyaux;

une plaque porte-chambres et porte-échantillons, formée de 3 creux pour la mise en place des récipients des chambres de réaction (12) et 4 creux pour la mise en place des récipients pour les échantillons et les controles (13);

récipients pour les chambres de réaction (12); chaque récipient contient 10 rangées de 15 chambres de réaction;

récipients porte-échantillons; chaque récipient prévoit 10 positions indifféremment pour petits verres de 1 ml ou de 2 ml;

récipient porte-controles; chaque récipient prévoit 10 positions pour petits verres de 1 ml;

cuvette de décharge des solutions de lavage des tuyaux;

cuvette pour la mise en place des réactifs et de la solution de lavage (14);

récipient pour les solutions de déchet;

imprimante.

L'usager a la possibilité d'enregistrer à l'aide d'un Programme dédié, les informations relatives au patient à analyser et de demander, en parcourant un panneau préparé, les analyses relatives à chaque patient. Une fois terminée l'introduction des données pour chaque patient, le Programme présente un projet de travail et indique comment positionner les récipients porte-chambres de réaction dans les plaques porte-chambres. Le Programme demande s'il est nécessaire d'exécuter le recalibrage des courbes standard et en cas affirmatif il demande d'insérer les petits verres relatifs dans les positions préétablies. Une fois achevée la phase préparatoire indiquée et après que l'on a appuyé sur la touche d'envoi analyse, l'instrument commence le procédé. Celui-ci prévoit la distribution des échantillons, l'attente des temps de réaction opportuns, les lavages et la lecture des résultats, après mise au zéro sur chaque chambre de réaction. A la fin du procédé d'analyse, le Programme compare chaque donnée obtenue à la courbe standard mémorisée pour le groupe auquel l'analyte demandé appartient, et il calcule les valeurs en Unités Relatives et Classes de positivité. A l'aide de l'imprimante on reproduit les fiches de réponse relatives à chaque patient, portant les données du patient et les résultats obtenus pour chacune des analyses demandées, résultats qui sont indiqués et en Unités Relatives et en Classes de positivité.

Les données relatives à chaque patient et l'analyse effectuée sont mémoriseés dans les archives présentes dans le Programme et elles peuvent aussi etre envoyées à l'aide d'une opportune connexion à l'ordinateur central de l'usager.

Les chambres de réaction présentent une forme de "U" de dimensions capillaires. Chaque chambre présente un bras vertical de diamètre plus petit (15) et un bras vertical plus grand (16), joints par un bras horizontal de base. Les chambres peuvent se présenter en groupes de 5 ou bien individuellement.

Chaque chambre est formée d'une partie supérieure en polystyrène luisant (17) et d'un bouchon en polyéthylène opalin de couleurs différentes suivant les groupes de molécules (18). La composition du matériel des chambres peut changer pour exigences de production.

La forme de la chambre a été dessinée comme décrit pour consentir la distribution des échantillons et des réactifs sans formation de bulles et le lavage soigné des fluides non réactifs. Elle peut éventuellement contenir des parties solides si celles-ci représentent un avantage qualitatif dans la réaction.

Le volume d'échantillon ou réactif contenu dans les chambres est compris entre 20 et 50 ul.

Le système indicateur comprend:

Un réactif convenable contenant l'anticorps conjugué à l'enzyme, comme par exemple Uréase. L'anticorps est spécifique pour l'analyte examiné. L'anticorps peut etre représenté indifféremment par des protéines monoclonales ou polyclonales et l'enzyme peut etre modifiée pour exigences de production et qualité; à la place de l'enzyme il est possible d'utiliser des molécules différentes pourvues de leur signal direct décelable (par exemple iode-125 à émission radioactive ou substances fluorescentes ou chimioluminescentes);

le réactif de relèvement constitué d'un substrat convenable pour l'enzyme utilisée. Dans le cas de l'Uréase il s'agit de pourpre de brome crésol et urée. La présence de l'Uréase détermine la trasformation de l'urée et par conséquent la modification du pH. La variation du pH détermine le virage de la couleur de l'indicateur de pH, pourpre de brome crésol, du jaune au violet, variation qui est mesurée par la tete de lecture. Le substrat peut changer en relation à l'enzyme utilisée, ou peut etre tout à fait absent au cas où l'indicateur serait marqué par une molécule à émission directe de signal (par exemple RIA, fluorescent ou chimioluminescent);

la solution de lavage constituée par un tampon et un détergent;

Le système de référence est formé par des sérums standard à concentration connue d'analyte pour concentrations différentes ( d'un minimum de 3 à un maximum de 8).

Il y a une courbe standard pour chaque groupe homogène de molécules en analyse et les sérums sont calibrés contre les standard internationaux de chaque groupe, au cas où ceux-ci auraient été déposés auprès des organisations légales responsables (par exemple le WHO de Genève).

Les calibreurs sont constitués par des sérums standard à concentration connue d'analyte.

Le système prévoit aussi l'introduction du Programme de Controle de Qualité Interlaboratoire.

**Revendications**

1. Moyen pour la détermination complètement automatique de molécules et immunoglobulines directes contre d'autres molécules (anticorps ou allergènes) dans des échantillons aqueux, comprenant chambres de réaction (17 et 18) auxquelles sont collés les anticorps spécifiques ou d'autres molécules spécifiques ou dans lesquelles on a inséré des segments auxquels ces molécules sont attachées, un premier réactif comprenant des anticorps directs contre les analytes à déterminer, anticorps rendus capables d'émettre un signal direct, ayant été marqués à l'aide d'un émetteur de radiations ou d'une autre énergie décelable, ou indirect, ayant été marqués par une enzyme capable de développer une réaction décelable avec un substrat opportun; ce premier réactif est destiné à rentrer en contact, in vitro, avec l'échantillon d'essai aqueux que l'on a déjà fait réagir dans les chambres de réaction appropriées. Dans le complexe s'étant formé entre la phase solide avec les molécules spécifiques attachées, des substances éventuellement attachées à celle-ci et la phase liquide irradiante ou marqués avec l'enzyme, la quantité de signal émise est fonction de la concentration d'analyte examinée dans la solution aqueuse testée et un deuxième réactif facultatif en condition d'expliciter le signal donné par la présence de l'enzyme.

2. Moyen de la revendication 1. où les chambres capillaires de réaction ont une forme spéciale de "U" (17, 18, 15, 16).

3. Moyen de la revendication 1. et 2. où les chambres capillaires de réaction en "U" sont individuelles.

4. Moyen de la revendication 1. et 2. où les chambres capillaires de réaction sont en groupes de 5.

5. Moyen de la revendication 1. et 2. où les chambres capillaires de réaction contiennent des segments réactifs.

6. Moyen de la revendication 1.,2.,3., et 4. où les chambres capillaires de réaction ont des molécules spécifiques attachées à la surface.

7. Moyen de la revendication 1.,2. et 5. où les segments contenus dans les chambres de réaction ont des molécules spécifiques attachées à la surface.

8. Moyen des revendications 1.,2.,6. et 7. où les molécules sont attachées aux surfaces et passivement et de facon covalente.

9. Moyen des revendication 1.,2. et 4. où toutes les 5 chambres de réaction contiennent les memes molécules attachées à la surface.

10. Moyen des revendication 1.,2. et 4. où chacune des 5 chambres contient une molécule différente attachée à la surface.

11. Méthode pour la détermination et quantification des analytes en solutions aqueuses et fluides biologique grace à l'immunodosage où on utilise les moyens des revendications 1.,2.,3.,4.,5.,6.,7.,8., 9.,10. de facon absolument automatique à l'aide d'un instrument capable de procéder à tous les passages nécessaires pour atteindre le résultat. La méthode comprend: remplissage de la chambre capillaire en "U" avec l'échantillon (6), attente que l'analyte dans l'échantillonn réagisse avec les molécules spécifiques attachées aux surfaces réactives, levée de l'échantillon de la dite chambre capillaire de réaction (5, 11), lavage intérieur de la dite chambre de réaction pour enlever les composants non spécifiquement liés aux surfaces réactives (4, 5, 10) remplissement de la dite chambre de réaction avec un deuxième réactif capable de réagir avec un des réactifs ou échantillons precedents (6), et précisément l'analyte présent dans l'échantillon ou le premier réactif lié aux susdites surfaces réactives, attente que la réaction impliquant le deuxième réactif puisse avoir lieu, levée du deuxième réactif de la dite chambre capillaire de réaction (5, 11), lavage intérieur de la dite chambre capillaire de réaction pour enlever le deuxième réactif ne s'étant pas spécifiquement lié (4, 5, 10), détermination de la quantité de ce deuxième réactif spécifiquement lié aux surfaces réactives (8).

**12.** Méthode dont à la revendication 11. où le susdit deuxième réactif est marqué à l'aide d'une molécule radioactive, fluorescente ou luminescente et la quantité de ce deuxième réactif spécifiquement liée aux surfaces réactives peut etre mesurée en comptant la quantité de radioactivité, fluorescence ou luminescence émises à travers la dite chambre capillaire de réaction.

**13.** Méthode dont à la revendication 11. où le susdit deuxième réactif est marqué à l'aide d'une enzyme et la quantité de ce deuxième réactif spécifiquement liée aux surfaces réactives peut etre déterminée, après adjonction à la susdite chambre capillaire de réaction d'un substrat, en mesurant la quantité de produit s'étant formé par catalyse enzymatique.

**14.** Méthode dont aux revendications 11.,12. et 13. où la mesure finale est quantitative, étant confrontée avec une courbe standard spécifique pour chaque groupe de molécules analysées, où chaque sérum standard est calibré contre le standard international de son groupe, au cas où celui-ci existerait déposé auprès des organisations internationales compétentes.

**15.** Moyen dont aux revendications 11.,12.,13. et 14., capable d'exécuter automatiquement tous les passages de réaction nécessaires sans aucune intervention de la part de l'opérateur.

**16.** Moyen dont aux revendications 11.,12.,13., et 14. capable d'exécuter automatiquement tous les passages de réaction nécessaires sans aucune intervention de la part de l'opérateur sauf que pour la lecture finale.

FIG. 1

FIG.2

FIG. 3

FIG. 4

FIG.5

FIG. 6